# EUROPEAN PATENT SPECIFICATION

(11) **EP 2 182 890 B1**
(45) Date of publication and mention of the grant of the patent: **12.12.2012**
(21) Application number: 08829447.5
(22) Date of filing: 30.07.2008
(51) Int. Cl.: D04B 21/12, A61L 27/38, A61L 27/56, A61L 27/58

(54) **BIORESORBABLE KNIT**
BIOLOGISCH ABBAUBARE MASCHENWARE
TRICOT BIORÉSORBABLE

(30) Priority: 30.07.2007 US 881837
(43) Date of publication of application: 12.05.2010
(73) Proprietor: SOFRADIM PRODUCTION, 01600 Trévoux (FR)
(72) Inventor: BAYON, Yves, F-69007 Lyon (FR); GRAVAGNA, Philippe, F-69540 Irigny (FR); MENEGHIN, Alfredo, F-69009 Lyon (FR); THERIN, Michel, F-69004 Lyon (FR); MONTANARI, Suzelei, F-01600 Trevoux (FR)
(74) Representative: Maureau, Philippe
(86) International application number: PCT/IB2008/002888
(87) International publication number: WO 2009/031035

(56) References cited:
- EP-A- 1 216 718
- WO-A-2009/016518
- WO-A-2009/016519
- WO-A-2009/031047
- US-A1- 2003 225 355
- US-A1- 2004 138 762
- US-A1- 2007 032 805

## Description

### TECHNICAL FIELD

The present disclosure relates to a bioresorbable three-dimensional knit that can be used as a bioresorbable wall reinforcement implant when a permanent implant is not necessary. The knit according to the present disclosure can be used in vitro as a tissue engineering product or as a support for culturing live cells.

### BACKGROUND

A hernia causes a defect in a wall of the human body, for example in the abdominal wall. Various other phenomena can create various faults, i.e. a lack of tissue, in various walls of the human body, for instance the visceral walls (intestine, stomach, uterus, bladder, urethra, ureter, etc.) and the abdominal wall.

In order to treat the drawbacks associated with these phenomena, wall reinforcement implants have been developed, for example based on a biocompatible textile which is implanted at the defect in order to overcome a lack of tissue. These implants are often permanent.

In order to limit the introduction of synthetic foreign bodies into the human body, implants have also been developed which are based on products obtained from porcine dermis or from a human cadaver, which are decellularized and then implanted at the wall defect. However, although these products are washed, they can cause necroses and death of the neighbouring tissues.

However, in certain cases, permanent implants are not necessary. Moreover, as indicated above, in the case of the treatment of these defects, one seeks to limit the amount of foreign bodies called upon to remain permanently in a human body and to promote tissue reconstruction.

Thus, it is preferable for the structure of the implant to be favourable to cell growth. At the same time, the implant must exhibit a minimum amount of mechanical strength in order to perform its reinforcement function. In particular, when the implant is bioresorbable, it is important for the cell colonization to take place gradually and in a controlled manner, and at the same time in a homogeneous manner, as the implant degrades.

Bioresorbable wall reinforcement implants already exist.

Thus, document US2003/0225355 describes an implant based on a bioresorbable collagen matrix that can trap a two-dimensional textile that may be bioresorbable. However, such an implant does not allow satisfactory cell growth. In particular, such an implant does not allow gradual, controlled and homogeneous cell colonization of the textile.

Document EP 1 216 718 describes an implant comprising a bioresorbable polymeric sponge reinforced with a two-dimensional textile. However, such an implant does not allow satisfactory cell growth either. In particular, such an implant does not allow gradual, controlled and homogeneous cell colonization of the textile.

Document US 6,262,332 describes a biomaterial comprising a layer of nonhuman collagen and a two-dimensional textile. However, such an implant does not allow satisfactory cell growth. In particular, such an implant does not allow gradual, controlled and homogeneous cell colonization of the textile.

Document US2007/0032805 discloses a three-dimensional fabric having a resorbable dense face that may be made of oxidized regenerated cellulose.

Thus, there remains the need for an entirely bioresorbable implant which has sufficient mechanical properties while at the same time allowing effective, gradual and controlled cell growth, so that the tissue regeneration is accomplished effectively during the time the implant is effectively present in the human body, i.e. before bioresorption of the implant.

### SUMMARY

The present disclosure aims to remedy this need by providing a bioresorbable three-dimensional prosthetic knit having a first face and a second face, the first face and the second face being opposite and separated from one another by the thickness of the knit and being connected to one another by a spacer. The first face and the second face are porous and comprise yarns made of material which undergoes slow bioresorption as mentioned in claim 1. The spacer includes yarns made of material which undergoes slow bioresorption and yarns made of material which undergoes rapid bioresorption, as mentioned in claim 1.

The knit according to the present disclosure can be directly used as an implant.

In the present application, the term "implant" is intended to be a biocompatible medical device that can be implanted into the human or animal body.

In the present application, the term "bioresorbable" is intended to mean the characteristic according to which a material is absorbed by biological tissues and disappears in vivo after a given period, which may vary, for example, from one day to several months, depending on the chemical nature of the material. For a material that has undergone a crosslinking step, the period of time necessary for the material to be absorbed in vivo will also depend on the degree of crosslinking of the material.

In the present application, the expression "yarn made of material which undergoes slow bioresorption" is intended to mean a yarn obtained from a material that can be completely bioresorbed or degraded in vivo, i.e. in the human body, according to an adaptable and controllable time period ranging from approximately 6 months to 2 years.

In the present application, the expression "yarn made of material which undergoes rapid bioresorption" is intended to mean a yarn which can be completely bioresorbed or degraded in vivo, i.e. in the human body, according to an adaptable and controllable period of time ranging from approximately 1 week to 6 months.

For the purpose of the present application, the term "porous" is intended to mean the characteristic according to which a structure exhibits pores, or alternatively gaps, alveoli, holes or orifices, which are open, which may or may not be evenly distributed, and which promote all cell colonization.

For the purpose of the present application, the term "three-dimensional knit" is intended to mean an assembly or arrangement of monofilament or multifilament yarns, obtained by knitting and having a significant thickness, in embodiments of greater than or equal to 0.5 mm. In embodiments, the yarns of the three-dimensional knit of the present disclosure are biocompatible.

In the present application, the term "spacer" is intended to mean the sheet(s) of yarns which connect(s) the two faces of a three-dimensional knit to one another, thus constituting the thickness of such a knit.

The knit according to the present disclosure, once implanted, exhibits at least two-speed bioresorption kinetics, with part of its structure being resorbed more rapidly than the other part. Such an embodiment thus makes it possible to create spaces gradually and in a controlled manner, in particular at the level of the spacer and therefore at the heart of the knit, that the cells will little by little colonize as the part made of material which undergoes rapid bioresorption degrades. The cell growth will thus take place in a controlled, gradual and homogeneous manner.

In embodiments of the present disclosure, said first face and said second face consist of yarns made of material which undergoes slow bioresorption.

In embodiments of the present disclosure, the yarns made of material which undergoes rapid bioresorption define a first network of yarns. This first network of yarns has a first density.

In embodiments of the present disclosure, the yarns made of material which undergoes slow bioresorption define a second network of yarns. This second network of yarns has a second density, which may be different from that of the first network of yarns. This second network of yarns, by decreasing the free space within the knit, increases the overall density of the knit.

In embodiments, the first network of yarns and the second network of yarns form pores, or alternatively channels, which are interconnected with one another.

For the purpose of the present application, the term "interconnected pores" is intended to mean open pores which are connected to one another and communicate with one another over the knit as a whole, without partitioning, such that a cell that is in a pore can pass from one pore to the other, over the entire knit, and can in theory circulate through all the pores of the knit.

For the purpose of the present application, the term "interconnectivity" is intended to mean the ability of the knit to allow any cell that is in a pore to circulate within all the other pores of the knit. Thus, in embodiments, in the knit according to the present disclosure, all the pores of the knit are accessible to any cell originating from the organism into which the knit is implanted.

Such a knit according to the present disclosure is particularly suitable for the treatment of wall defects and for tissue reconstruction when a permanent reinforcement is not necessary. In fact, due to its bioresorbable three-dimensional porous structure in which all the pores are interconnected, such a knit according to the present disclosure promotes a gradual, controlled and homogeneous cell growth. Thus, as each element of the knit, i.e. the yarns made of material which undergoes rapid bioresorption of the spacer, and then the yarns made of material which undergoes slow bioresorption of the knit, degrade in vivo, the cells proliferate and regenerate the organic tissue at the site of the defective wall. The more the regenerated organic tissue grows, the more the mechanical strength of the knit decreases, subsequent to its gradual degradation. In addition, the cells can circulate through all the sites of the knit according to the present disclosure by virtue of the interconnectivity of the pores defined by the yarns made of material which undergoes slow bioresorption and by the yarns made of material which undergoes rapid bioresorption: thus, the cell growth is evenly distributed over the entire knit, leaving, once the knit is completely resorbed, an organic tissue reconstructed at the site where the knit was initially implanted, i.e. at the site of the original tissue defect.

Thus, the degradation of the yarns made of material which undergoes rapid bioresorption creates, within the knit according to the present disclosure, new pores or channels, which are interconnected with the pores or channels of the knit that are defined by the yarns made of material which undergoes slow bioresorption, and the cell growth can become distributed in a homogeneous, gradual and controlled manner, and can little by little invade the space left free by the degradation of the material which undergoes rapid in vivo bioresorption. However, during the degradation of the material which undergoes rapid in vivo bioresorption, the knit maintains sufficient mechanical strength due to the presence of the material which undergoes slow in vivo bioresorption, the three-dimensional network of which gives the knit most of its mechanical properties.

The yarns made of material which undergoes slow bioresorption is poly(lactic acid) (PLA).

The yarns made of material which undergoes rapid bioresorption is oxidized cellulose.

Among the yarns made of material which undergoes rapid bioresorption, certain can be partially degraded, in order to increase their rate of bioresorption and/or to adjust their biodegradation time. For example, the yarns made of poly(lactic acid) can be partially degraded by a treatment such as repeated cycles of gamma-irradiation at doses greater than or equal to 25 kGy, until the desired degradation time is obtained.

The yarns which undergo rapid bioresorption can also be made of oxidized cellulose. These yarns can be obtained by any method for oxidizing cellulose known to those skilled in the art.

In embodiments, the oxidized celluloses are chosen from oxidized cellulose in which the primary alcohol at C₆ is partially or completely oxidized to carboxylic acid, for example to give poly(glucuronic acid), cellulose oxidized in the form of polyaldehydes by periodic acid, "viscose"-type cellulose, manufactured from a paste of cellulose that has been solubilized, and then regenerated and oxidized, and mixtures thereof.

Several varieties of regenerated cellulose have been industrially developed. Mention may, for example, be made of the "viscose" process which is based on the solubility of cellulose xanthate in a dilute solution of sodium hydroxide. Mention may also be made of the process referred to as "cupro-ammonium process", used for example by Bemberg S.p.A., Gozzano, Italy and Asahi Kasei Fibers Corporation, Tokyo, Japan, and which consists in dissolving the cellulose in an ammoniacal solution of copper. Another process for preparing regenerated cellulose suitable for the present disclosure is the process of dissolving cellulose in an organic phase with N-methylmorpholine oxide (N.M.M.O.), referred to as "Lyocell® process", used, for example, by Lenzing Aktiengesellschaft, Austria.

When spun through a perforated plate, the viscose coagulates in acidic medium and forms long continuous filaments of regenerated cellulose, which are dried and combined into multifilament yarns. A yarn of regenerated cellulose having good mechanical strength is obtained.

In general, such a yarn of regenerated cellulose is not resorbable. Thus, as will be described later in the present application, the knit according to the present disclosure will in embodiments, firstly, be made with such a yarn of regenerated cellulose, and then, secondly, the knit will be subjected to an oxidation process in order to render the yarn of regenerated cellulose bioresorbable.

By way of example, as yarn made of material which undergoes rapid bioresorption, mention may be made of yarns made of regenerated and oxidized cellulose.

The yarns made of material which undergoes slow bioresorption and the yarns made of material which undergoes rapid bioresorption that are used to produce the knit according to the present disclosure can be multifilament yarns or monofilament yarns or a combination thereof.

In embodiments of the present disclosure, the yarns made of material which undergoes slow bioresorption that are used for the spacer are monofilament yarns. The monofilament yarns made of material which undergoes slow bioresorption, located in the spacer, make it possible to maintain the thickness or alternatively the spacing between the two porous faces of the knit. The presence of monofilament yarns in the spacer makes it possible to confer excellent mechanical strength on the knit according to the present disclosure. In particular, in the optional step of thermosetting the knit, the knit maintains its mechanical properties intact. The knit can thus be handled by the surgeon extremely easily. Moreover, such a knit effectively performs its wall reinforcement functions throughout the period necessary for cell colonization in order to regenerate the tissue at the site of the original tissue defect and in the three-dimensional space provided by the knit.

In embodiments of the present disclosure, the yarns made of material which undergoes rapid bioresorption are multifilament yarns. Thus, the presence of multifilament yarns in the spacer makes it possible to increase the density of the latter. The degradation of these multifilament yarns made of material which undergoes rapid bioresorption will make it possible, after implantation, to generate new pores or tunnels that promote the development of cell growth.

The presence of multifilament yarns in the spacer makes it possible to vary the density and therefore the porosity of the knit according to the present disclosure.

Thus, in embodiments, the spacer is made of a combination of monofilament yarns and multifilament yarns.

The interconnectivity of the pores can also be controlled, to a certain extent, by the density of the spacer yarns and their distribution between the two faces of the three-dimensional knit. Similarly, the density and the porosity of the knit according to the present disclosure vary according to the density of the yarns used. Thus, by increasing the density of the yarns used, the three-dimensional porosity of the knit according to the present disclosure is reduced.

Thus, in embodiments, the count of the monofilament yarns made of material which undergoes slow bioresorption ranges from 200 to 500 dtex.

A monofilament yarn made of material which undergoes slow resorption that is suitable for the present disclosure is, for example, a 220 dtex monofilament yarn with a diameter of approximately 150 µm, made of poly(lactic acid).

The count of the multifilament yarns made of material which undergoes rapid bioresorption can range from 50 to 300 dtex, in embodiments from 80 to 220 dtex.

A multifilament yarn made of material which undergoes rapid bioresorption that is suitable for the present disclosure is, for example, the 90 dtex multifilament yarn made of regenerated cellulose, sold under the name "CUPRO® Cusio" by the Italian company Bemberg, oxidized so as to make it bioresorbable.

In embodiments of the implant of the present disclosure, the knit has a two-dimensional porosity of less than or equal to 30%, preferably of less than or equal to 20%.

For the purpose of the present application, the term "two-dimensional porosity" is intended to mean a porosity calculated from two-dimensional images corresponding to views from above the knit according to the present disclosure, these images then being processed by software which analyses them, for instance the Image J software.

In embodiments of the present disclosure, the second network of yarns made of material which undergoes slow bioresorption, also called the ground, has a three-dimensional porosity of greater than or equal to 80%, preferably greater than or equal to 85%, and more preferably greater than or equal to 90%.

For the purpose of the present application, the term "three-dimensional porosity" is intended to mean a porosity measured in the following way: the dimensions, i.e. length, width and thickness, of the ground made of yarns made of material which undergoes slow bioresorption of the knit, taken alone, are measured; moreover, the density of the yarns used to knit this ground are known. The ground is weighed. By means of a simple subtraction, the volume occupied by the empty spaces within the ground is deduced therefrom. The three-dimensional porosity over the entire ground is determined as being the percentage of empty volume relative to the total volume of the ground.

Thus, in embodiments, the knit according to the present disclosure has a two-dimensional porosity of less than or equal to 30%, preferably of less than or equal to 20% and the second network of yarns made of material which undergoes slow bioresorption has a three-dimensional porosity of greater than or equal to 80%, in embodiments greater than or equal to 85%, and in embodiments greater than or equal to 90%.

The three-dimensional porosity of the ground of the knit made of yarns made of material which undergoes slow bioresorption, according to the present disclosure, makes it possible to limit as much as possible the mass of slowly resorbable textile in the knit according to the present disclosure, and therefore the mass of foreign body that remains more than 6 months after its implantation.

Furthermore, it is also advantageous for the knit according to the present disclosure to have a relatively low two-dimensional porosity, in embodiments less than or equal to 30%, preferably of less than or equal to 20% in order to promote as much as possible the tissue integration and the cell colonization of the knit according to the present disclosure, by increasing its developed surface area.

In embodiments of the implant of the present disclosure, the three-dimensional knit has a thickness ranging from approximately 2 mm to 6 mm, preferably ranging from 2 mm to 4 mm.

The thickness of the three-dimensional knit defines the space in which the regeneration of the defective wall will take place. It is thus determined by the thickness of the wall to be regenerated. In embodiments, it is equivalent to the thickness of the wall to be regenerated.

In embodiments of the implant according to the present disclosure, the knit is isoelastic.

For the purpose of the present application, the term "isoelastic knit" is intended to mean a knit which has isotropic elastic mechanical properties, i.e. substantially equivalent in all directions.

In embodiments, the ratio of respective extensions in the warp direction and in the weft direction is between 0.4 and 2.5, at a physiological force of for example 50 N for abdominal wall repair.

It has been found that such an isoelastic knit allows excellent reinforcement of visceral walls. Specifically, the knit is deformed and extended in a more homogeneous manner, thus limiting the risk of wall or hernia rupture.

In embodiments of the knit of the present disclosure, at least a part of the yarns constituting the three-dimensional knit are coated with a bioresorbable coating. For example, the coating can be chosen from collagen, polysaccharides and mixtures thereof. The polysaccharides can be chosen from hyaluronic acid, alginic acid, poly(glucuronic acid), chitosan, starch, soluble cellulose derivatives, and mixtures thereof. Such a yarn coating makes it possible in particular to eliminate any possible crevice within the knit of the implant according to the present disclosure, for example where the yarns cross, such crevices being liable to create sites where bacteria or inflammatory cells develop. Such a knit thus makes it possible to reduce the risks of inflammation and sepsis, the bioresorbable coating making the accessible surface of the knit completely smooth and thus preventing installation of undesirable bacteria and/or microorganisms and/or inflammatory cells.

In embodiments of the present disclosure, the knit also includes one or more active compounds for improving wall and tissue repair. Illustrative examples of compounds for improving wall and tissue repair include antiseptics, anti-inflammatories, growth factors, extracellular matrix proteins such as fibronectin, laminin, elastin, glycosaminoglycans or proteoglycans, and mixtures thereof.

In embodiments of the present disclosure, the knit also includes a bioresorbable film on at least one of its faces. The film can include at least collagen. The film can, for example, include oxidized collagen, polyethylene glycol and glycerol. Such a film preferably has a smooth anti-adhesive surface and is particularly suitable for the manufacture of a knit that can be used as a wall reinforcement implant that also has anti-adhesive properties.

The knit of the present disclosure may undergo an oxidation step. Thus, the process for preparing the knit of the present disclosure can include a first knitting step for manufacturing the knit, and then a subsequent step consisting of oxidation of the knit. In such a case, it is possible to choose, as yarn made of material which undergoes rapid bioresorption, a yarn which is nonbioresorbable before oxidation, and bioresorbable after oxidation. This is, for example, the case when a yarn made of regenerated, for example nonoxidized, cellulose is chosen as yarn made of material which undergoes rapid bioresorption. The yarn made of regenerated nonoxidized cellulose becomes bioresorbable after an oxidation step.

Another aspect of the present disclosure is a process for manufacturing a knit as described above in which at least part of the yarns made of material which undergoes rapid bioresorption are made of oxidized cellulose, that includes at least the following steps:
- a°) the knit is produced on a knitting machine with yarns, at least part of the yarns being made of nonoxidized cellulose,
- b°) the knit produced in step a°) is submitted to an oxidation step.

In an embodiment of the present disclosure, the oxidation step is carried out with NO₂.

In another embodiment of the present disclosure, the oxidation step is carried out with sodium periodate.

In embodiments of the present disclosure, the knit is seeded with live cells. Thus, the present disclosure also relates to a tissue engineering support, characterized in that it includes at least one knit as described above. This support can be seeded with live cells.

The present disclosure also relates to the use of a knit or of a support as described above, for culturing live cells.

### BRIEF DESCRIPTION OF THE DRAWINGS

Embodiments of the present disclosure will be described more clearly by means of the description which follows and the attached drawings in which:
- Figures 1 to 3 represent patterns of knits suitable for the implant according to the present disclosure,
- Figures 4 and 5 represent scanning electron microscopy images (Hitachi S800 scanning electron microscope with image acquisition and analysis system) respectively of one face and of the spacer of the knit according to Figure 1,
- Figures 6 and 7 represent scanning electron microscopy images (Hitachi S800 scanning electron microscope with image acquisition and analysis system) respectively of one face and of the spacer of the knit according to Figure 2,
- Figures 8 and 9 represent scanning electron microscopy images (Hitachi S800 scanning electron microscope with image acquisition and analysis system) respectively of one face and of the spacer of the knit according to Figure 3.

### DETAILED DESCRIPTION OF PREFERRED EMBODIMENTS

The knit according to the present disclosure can consist of monofilament and/or multifilament threads made of bioresorbable material.

The knit according to the present disclosure includes a first face and a second face, opposite and separated from one another by the thickness of the knit. The first and second faces are preferably connected to one another by a spacer. For example, the spacer consists of a sheet of linker yarns. Each face can consist of one or more sheets of yarns.

In one embodiment of the present disclosure, the first and second faces of the knit are identical. For example, each face consists of two sheets of yarns. In embodiments, the yarns constituting the two faces of the knit are made of multifilament yarns of poly(lactic acid). Such yarns resorb completely in vivo in the space of 6 months to 2 years. Yarns suitable for producing the two faces of the knit of the implant according to the present disclosure are, for example, 84 dtex poly(lactic acid) multifilament yarns with 24 filaments per yarn, each filament having a diameter of approximately 18 µm.

In one embodiment of the present disclosure, the yarns constituting the spacer are a combination of monofilament yarns made of material which undergoes slow bioresorption and multifilament yarns made of material which undergoes rapid bioresorption. Such an embodiment makes it possible to confer on the knit excellent mechanical strength and excellent resistance to thermosetting when the knit is thermoset after the knitting phase. In embodiments, the spacer includes monofilament yarns made of material which undergoes slow bioresorption, made of poly(lactic acid). Such yarns suitable for preparing the spacer of the knit of the implant according to the present disclosure are, for example, 220 dtex poly(lactic acid) monofilament yarns, the monofilament having a diameter of approximately 150 µm. In embodiments, the spacer includes multifilament yarns made of material which undergoes rapid bioresorption, made of oxidized regenerated cellulose.

The knit according to the present disclosure can be produced on a knitting machine of the Raschel type, for example using 5 or 6 bars, with a gauge of 22.

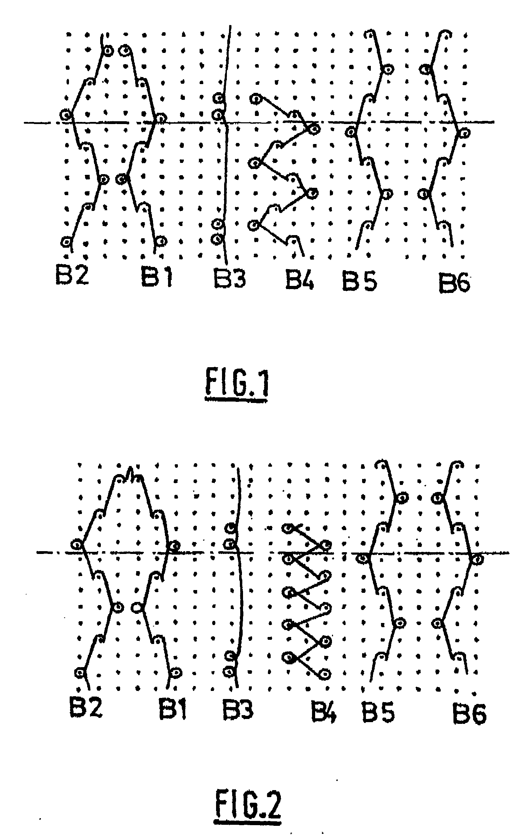

Examples of patterns suitable for the knit of the implant according to the present disclosure are shown in Figures 1 to 3.

In these figures, the references B1-B6 represent the bars 1 to 6.

The first face can, for example, be produced with bars 1 and 2. The second face can be produced in the same way, with bars 5 and 6.

The spacer can be produced with bars B3 and B4. Spacer bars B3 and B4 can be threaded as 1 and 1 complementary threading or as 2 and 1, 3 and 1, 4 and 1 complementary threading. The term "1 and 1 complementary threading" is intended to mean the bars are threaded 1 full, 1 empty, the full of one being opposite the empty of the other. According to a 2 and 1 complementary threading, one bar is threaded 2 full, 1 empty and the other bar is threaded 2 empty, 1 full.

Once the knit has been produced, it can undergo an oxidation step. Thus, the process for preparing the knit of the present disclosure can include a first knitting step for manufacturing the knit, and then a subsequent step consisting of oxidation of the knit. In such a case, it is possible to choose, as yarn made of material which undergoes rapid bioresorption, a yarn which is nonbioresorbable before oxidation, and bioresorbable after oxidation. This is, for example, the case when a yarn made of regenerated, for example nonoxidized, cellulose is chosen as yarn made of material which undergoes rapid bioresorption. The yarn made of regenerated nonoxidized cellulose becomes bioresorbable after an oxidation step.

The yarns made of material which undergoes rapid bioresorption of the knit according to the present disclosure define first pores, or channels, alveoli, gaps, holes or orifices. These first pores are interconnected with one another and with the second pores or alveoli, gaps, holes or orifices defined by the yarns made of material which undergoes slow bioresorption of the knit according to the present disclosure. The porosity and/or the density of the first network of yarns which undergo rapid bioresorption is preferably controlled by the threading of the yarns on the bars of the knitting machine, by the count of the yarns used and by the gauge of the knitting machine.

All the pores and/or gaps, for instance channels, created by the knitting at each face of the knit and in the thickness of the knit are open, connected to one another and communicate with one another: for example, it is possible for a cell to pass from one pore to the other, over the entire knit according to the present disclosure. All the pores are thus interconnected.

The pores of the knit according to the present disclosure define, for the knit, a two-dimensional porosity and, for the second network of yarns which undergo slow bioresorption, a three-dimensional porosity.

In the present application, the two-dimensional porosity is calculated from two-dimensional images corresponding to views from above the knit according to the present disclosure, these images then being processed by software which analyses them, for instance the Image J software. For example, for a measurement, the density of the knit was determined using a Nikon SMZ 800 binocular microscope with a Nikon DN100 digital camera used in combination with a PC computer. The digital images seen from above the knit were multiplied by a factor of 20 and were then processed by the Image J software in order to determine the density of the knit. Once the digital image is captured by the software, it is processed such that the surface area corresponding to the empty spaces in the knit is subtracted from the total surface area of the image. The two-dimensional porosity is determined as being the percentage corresponding to the rest of the digital image.

In one embodiment, the knit of the implant according to the present disclosure has a two-dimensional porosity, measured as indicated above, of less than or equal to 30%, preferably of less than or equal to 20%.

In the present application, the three-dimensional porosity is calculated as follows: the dimensions, i.e. length, width and thickness of the ground of yarns which undergo slow bioresorption of the knit, taken alone, are measured; moreover, the density of the yarns used to knit this ground is known. The ground is weighed. The volume occupied by the empty spaces within the ground is deduced therefrom by simple subtraction. The three-dimensional porosity over the ground as a whole is determined as being the percentage of empty volume relative to the total volume of the ground.

In one embodiment, the ground of the knit made of yarns made of material which undergoes slow resorption according to the present disclosure has a three-dimensional porosity, measured as indicated above, of greater than or equal to 80%, in embodiments greater than or equal to 85%, and in embodiments greater than or equal to 90%. The overall three-dimensional porosity of the knit according to the present disclosure can then be adjusted by varying the density of the yarns used, in particular the density of the yarns made of material which undergoes rapid bioresorption.

Thus, in embodiments, the knit according to the present disclosure has a two-dimensional porosity of less than or equal to 30%, preferably of less than or equal to 20% and the second network of yarns which undergo slow bioresorption, or ground, has a three-dimensional porosity of greater than or equal to 80%, in embodiments greater than or equal to 85%, and in embodiments greater than or equal to 90%.

Furthermore, it is also advantageous for the knit of the implant according to the present disclosure to have a relatively low two-dimensional porosity, in embodiments less than or equal to 30%, preferably less than or equal to 20%, in order to promote the tissue integration and the cell colonization of the knit.

In one embodiment of the present disclosure, the three-dimensional knit has a thickness ranging from approximately 2 mm to 6 mm, in embodiments ranging from 2 mm to 4 mm.

In one embodiment of the present disclosure, the knit is isoelastic, i.e. it has isotropic elastic mechanical properties, i.e. substantially equivalent in all directions.

Thus, in embodiments, the knit according to the present disclosure has a mechanical strength in the longitudinal direction, i.e. in the direction of the warp of the knit, measured according to ISO standard 13934-1 (properties of substances in tensile testing), ranging from 50 to 300 N. In embodiments, the knit according to the present disclosure has a mechanical strength in the transverse direction, i.e. in the direction of the weft of the knit, measured according to ISO standard 13934-1, ranging from 50 to 300 N.

In embodiments, the knit according to the present disclosure has a mechanical strength in the longitudinal direction, i.e. in the direction of the warp of the knit, measured according to ISO standard 13934-1, ranging from 100 to 250 N. In embodiments, the knit according to the present disclosure has a mechanical strength in the transverse direction, i.e. in the direction of the weft of the knit, measured according to ISO standard 13934-1, ranging from 75 to 200 N.

In embodiments, the knit according to the present disclosure has an elongation at 50N in the longitudinal direction, i.e. in the direction of the warp of the knit, measured according to ISO standard 13934-1, ranging from 10% to 50%. In embodiments, the knit according to the present disclosure has an elongation at 50N in the transverse direction, i.e. in the direction of the weft of the knit, measured according to ISO standard 13934-1, ranging from 10% to 50%.

In one embodiment, at least part of the yarns constituting the knit are covered with a bioresorbable coating. The bioresorbable coating can be chosen from oxidized collagen, glutaraldehyde-crosslinked collagen, hexamethylenediisocyanate (HMDI) crosslinked collagen, diisocyanates cross-linked collagen, bifunctional or trifunctional glycidyl ethers crosslinked collagen, carbodiimides crosslinked collagen, acyl azides crosslinked collagen, divinylsulphone crosslinked collagen, collagen crosslinked by UV-, beta- or gamma-irradiation or by heat treatment, and mixtures thereof. All of the yarns constituting the knit may be covered with such a coating. For example, the coating is made of collagen. In particular, a collagen chosen from the group comprising oxidized collagen, glutaraldehyde-crosslinked collagen, hexamethylenediisocyanate (HMDI) crosslinked collagen and mixtures thereof can be used for such a coating.

In one embodiment, the yarns of the knit are at least in part covered by coating the knit in a solution or suspension of collagen, in one step or in several steps. A coating step includes the actual coating of the knit with the collagen and the drying of the knit. The collagen deposited on the yarns can be crosslinked with glutaraldehyde after each application, as many times as the total number of coating cycles. In another embodiment, the collagen deposited on the yarns can be crosslinked with hexamethylenediisocyanate (HMDI) after each application, as many times as the total number of coating cycles. In embodiments, the yarns are covered by carrying out two or three successive coating cycles.

In another embodiment, the bioresorbable coating can be chosen from polysaccharides including hyaluronic acid, alginic acid, poly(glucuronic acid), chitosan, starch, soluble cellulose derivatives and mixtures thereof.

In another embodiment, before it is coated with a bioresorbable coating described above, the knit according to the present disclosure can be subjected to a surface treatment in order to render it more hydrophilic and thus promote the deposition of the collagen and/or the polysaccharides mentioned above on the knit.

The surface treatment can be carried out according to any process known to those skilled in the art.

Such a coating makes it possible to reduce the surface area of the knit accessible to bacteria and to inflammatory cells. The risks of inflammation and sepsis are thus reduced.

In one embodiment of the present disclosure, the knit also includes one or more biological active agents that promote tissue regeneration, chosen, inter alia, from antiseptic agents, anti-inflammatory agents, growth factors, sulphated polysaccharides such as fucans, extracellular matrix proteins such as fibronectin, laminin or elastin, glycosaminoglycans, proteoglycans, and mixtures thereof. This active agent may, for example, be incorporated into the sponge during manufacture of the implant.

The knit according to the present disclosure can be knitted on a knitting machine of the Raschel type. This knit is, in embodiments, thermoset, for example by being placed in an oven at from 100 to 200°C, for 30 s to 5 minutes, depending on the chemical nature of the yarns used. The knit is then cut to the sizes desired for the implant. The thermosetting can also be carried out after the knit has been cut up.

The knit according to the present disclosure can also be coated, on at least one of its faces, with a bioresorbable film. In embodiments, this film is smooth on the surface and can be used for the prevention of post-surgical adhesions.

Such a film may be a collagen film. In one embodiment of the present disclosure, such a film includes oxidized collagen, polyethylene glycol and glycerol.

This bioresorbable film can be applied to one face of the knit according to the present disclosure in the following way: a solution, for example of oxidized collagen, polyethylene glycol and glycerol, is prepared and then spread out in order to form a thin sheet on a hydrophobic flat support, for example on a support of polyvinyl chloride or polystyrene. The face of the knit to be coated can then be applied carefully to the collagen gel. After exposure to ambient temperature and evaporation, a film which coats one face of the knit is obtained. It is also possible to coat the two faces of the knit with such a film. This film preferably resorbs rapidly in vivo, for example in less than 8 days.

Thus, the knit according to the present disclosure is entirely bioresorbable in vivo. It is, as a result, suitable for treatments, for example for parietal defects, that do not require a permanent reinforcement. By virtue of the gradual degradation of the various yarns constituting the knit, for example, firstly, the yarns made of material which undergoes rapid bioresorption, and then, secondly, the yarns made of material which undergoes slow bioresorption, this leaves more and more space for the cell growth, which takes place gradually, while, during this time, the knit conserves the mechanical properties required for its function by virtue of its ground made of yarns which undergo slow bioresorption. The more the mechanical strength of the knit decreases due to the gradual degradation of the yarns of which it is made up, the more the intrinsic strength of the treated wall increases due to the presence of regenerated tissue, this regenerated tissue invading and trapping little by little the remainder of the knit until the latter is completely resorbed.

The knit according to the present disclosure can also be used in vitro as a tissue engineering support for cell culture. Thus, it is possible to seed the knit according to the present disclosure with live cells. Such live cells, cultured within the knit according to the present disclosure, can release growth factors and extracellular matrix, which can have an important role in the repair and/or strengthening of soft tissues. Thus, it is possible to provide the knit according to the present disclosure, in vitro, with cells that promote tissue repair, and then to subsequently implant the knit into the wall of the soft tissue to be strengthened. The repair is thus accelerated in vivo due to the presence of cells promoting regeneration as soon as the knit is implanted.

The knit according to the present disclosure can be seeded with cells chosen from the following cells, alone or in any possible combinations thereof: striated muscle cells, smooth muscle cells, endothelial cells, epithelial cells, mesothelial cells, fibroblasts, myofibroblasts, and stem cells of each of the above cell types. As an example, the cells may be selected from the group consisting of striated muscle cells, smooth muscle cells, endothelial cells, epithelial cells, mesothelial cells, fibroblasts, myofibroblasts, stem cells of striated muscle cells, stem cells of smooth muscle cells, stem cells of endothelial cells, stem cells of epithelial cells, stem cells of mesothelial cells, stem cells of fibroblasts, stem cells of myofibroblasts, and combinations thereof.

For example, it is possible to seed the knit described above with striated or smooth muscle cells, with their progenitors, and fibroblasts, in order to obtain effective wall repair.

Moreover, it is also possible to use a knit as described above, one face of which is coated with a bioresorbable film: for example, muscle cells can be cultured within the sponge of the knit, while endothelial or epithelial cells are cultured on the bioresorbable film. These endothelial or epithelial cells, after implantation of the knit, make it possible to accelerate the formation of a new endothelium or epithelium in vivo.

Similarly, it is possible to carry out effective reconstruction of an abdominal wall by seeding, before implantation, a knit according to the present disclosure with mesothelial cells on the film and with striated muscle cells in the knit.

Similarly, it is possible to carry out effective reconstruction of a bladder by seeding, before implantation, a knit according to the present disclosure with urothelial cells on the film and with smooth muscle cells in the knit.

The present disclosure also relates to a method for repairing a wall defect, characterized in that it includes the step consisting in implanting a knit as described above, seeded or not, at the site of the wall defect.

The examples which follow illustrate embodiments of the present disclosure.

### EXAMPLES:

### EXAMPLE 1: Preparation of a knit according to the present disclosure

A three-dimensional knit is produced on a double needlebar Raschel knitting machine, with 6 guide bars. Each of the faces of the knit, i.e. the first face and the second face, is produced with two guide bars. With reference to Figure 1, the first face is produced with bars B1 and B2, and the second, opposite, face is produced with bars B5 and B6, each bar being threaded one full, one empty, with the following respective charts:

| | |
|---|---|
| Bar B1: | 1-0-1-1/1-2-2-2/2-3-2-2/2-1-1-1//. |
| Bar B2: | 2-3-2-2/2-1-1-1/1-0-1-1/1-2-2-2//. |
| Bar B5: | 2-2-2-1/1-1-1-0/1-1-1-2/2-2-2-3//. |
| Bar B6: | 1-1-1-2/2-2-2-3/2-2-2-1/1-1-1-0//. |

The pattern corresponding to bars 1, 2, 5 and 6 is reproduced in Figure 1. Such threading and such a pattern result in porous faces. It is possible to adapt the pattern so as to have alveoli or pores on each face, opposite one another or shifted with respect to one another, in order to make the three-dimensional knit more or less transparent.

Bars B1-B2 and B5-B6 which produce the first and second faces of the knit are threaded with 84*/24° multifilament yarns (decitex count: 84 g per 10 000 m/number of filaments) of poly(lactic acid). The filament diameter of the multifilament yarns is approximately 18 µm.

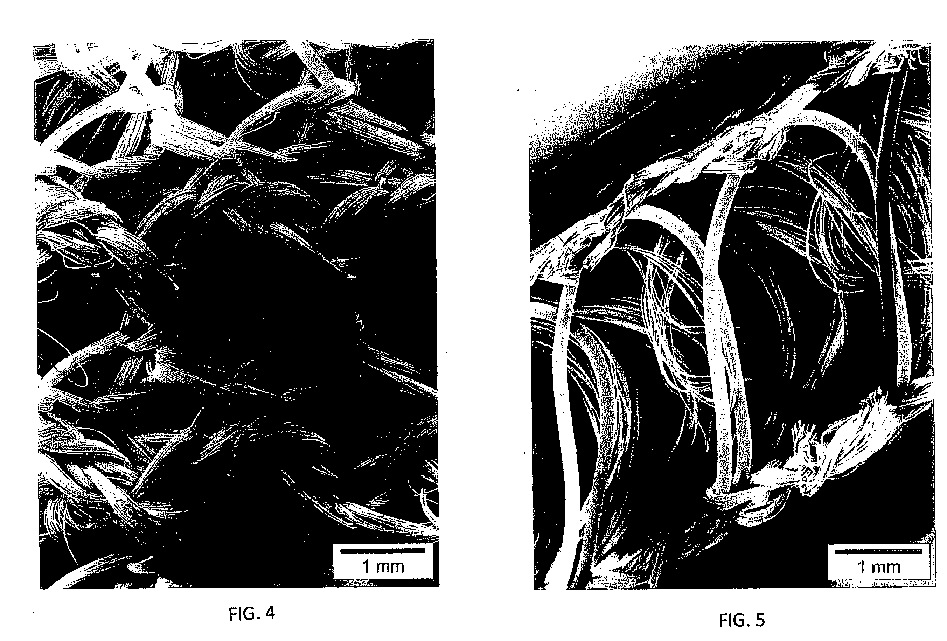

Figure 4 represents a scanning electron microscopy image of one face of such a knit.

With reference to Figure 1, the spacer is produced using bars B3 and B4, threaded one full, one empty, according to the following respective charts:

| | |
|---|---|
| Bar B3: | 0-1-0-1/0-0-0-0/0-0-0-0/0-0-0-0//. |
| Bar B4: | 1-2-3-4/3-2-1-0/1-2-3-4/3-2-1-0//. |

Bar B3 is threaded with 220 dtex monofilament yarns which have a diameter of approximately 150 µm, made of poly(lactic acid).

Bar B4 is threaded with 90 dtex multifilament yarns of regenerated cellulose, sold under the name "CUPRO^{®} Cusio" by Bemberg S.p.A., Gozzano, Italy.

The pattern used for the knitting is reproduced in Figure 1.

Figure 5 represents a scanning electron microscopy image of the spacer of such a knit.

Once the knit has been produced, it is thermoset by placing it in an oven at approximately 100°C for 1 to 5 min.

Such a knit has the following properties, measured as indicated in the present application:
Weight per surface area (g/m²): 250
Thickness: 4 mm
Porosity of the ground made of PLA: ≥ 80%
Two-dimensional porosity: < 20%

This knit is isoelastic. In particular, it has the following mechanical properties:

| Property | Str Wa | Str We | EI B Wa | EI B We | EI Wa 50 N | EI We 50 N |
|---|---|---|---|---|---|---|
| Knit Example 1 | 170 | 148 | 65 | 43 | 40 | 25 |

- Str Wa:: Mechanical Strength in the direction of the warp (in N); calculated according to ISO standard 13934-1
- Str We:: Mechanical Strength in the direction of the weft (in N); calculated according to ISO standard 13934-1;
- EI B Wa:: Elongation at break in the direction of the warp (as %) calculated according to ISO standard 13934-1;
- EI B We:: Elongation at break in the direction of the weft (as %) calculated according to ISO standard 13934-1;
- EI Wa 50 N:: Elongation at 50 N in the direction of the warp (as %) calculated according to ISO standard 13934-1;
- EI We 50 N:: Elongation at 50 N in the direction of the weft (as %) calculated according to ISO standard 13934-1.

In a further step, the knit it is subjected to an oxidation step with NO₂.

This oxidation is carried out by reacting NO₂ gas at a concentration of 10 g/I, in a proportion of 1.5 gram of NO₂ per gram of cellulose. The reaction is carried out for 4 hours. At the end of the reaction, washing with an inert gas such as CO₂ or N₂ is carried out in order to remove the excess NO₂. Washing with an isopropanol/water mixture (1:1) and then with pure isopropanol is then carried out.

The knit is subsequently vacuum-dried, and then cut up into the form of reinforcement prosthesis, which are packaged and sterilized with ethylene oxide.

According to this oxidation process, the multifilament yarns made of cellulose are oxidized and exhibit rapid in vivo bioresorption.

### EXAMPLE 2: Preparation of a knit according to the present disclosure

A knit is produced with the same threadings for bars B1 to B6 and according to the same process as in Example 1, with the difference that bar B4 has the following chart (see Figure 2):
- Bar B4:: 1-0-2-3/1-0-2-3/0-1-2-3/0-1-2-3//.

Bar B4 is threaded with 90 dtex multifilament yarns of regenerated cellulose sold under the name "CUPRO® Cusio" by Bemberg S.p.A., Gozzano, Italy.

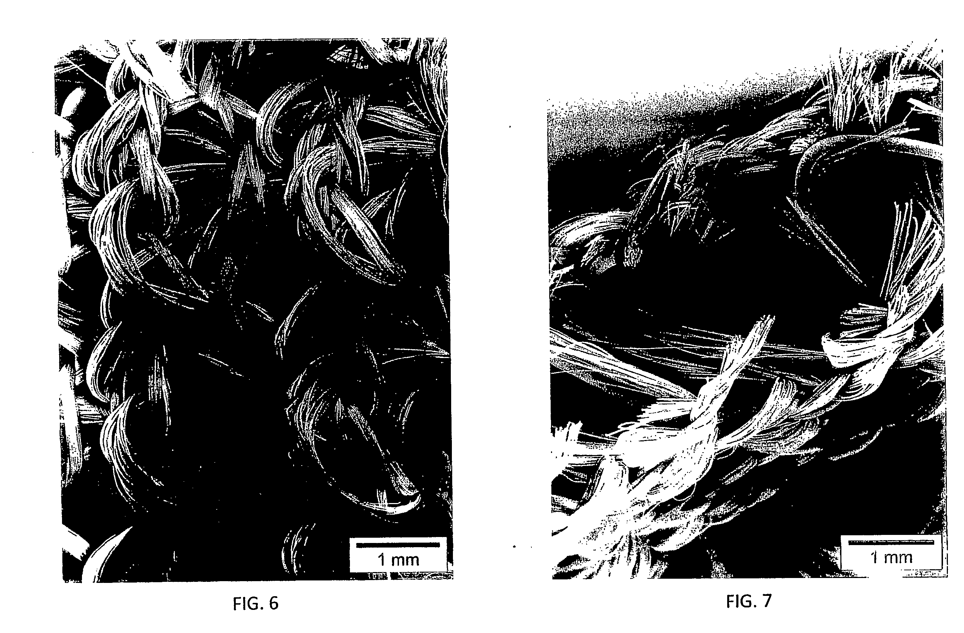

Figure 6 represents a scanning electron microscopy image of one face of such a knit and Figure 7 represents a scanning electron microscopy image of the spacer of such a knit.

Such a knit has the following properties, measured as indicated in the present application:
Weight per surface area (g/m²): 240
Thickness: 4 mm
Porosity of the PLA ground ≥ 80%
Two-dimensional porosity: < 20%

This knit is isoelastic. In particular, it has the following mechanical properties:

| Property | Str Wa | Str We | EI B Wa | EI B We | EI Wa 50 N | EI We 50 N |
|---|---|---|---|---|---|---|
| Knit Example 2 | 137 | 146 | 56 | 39 | 37 | 23 |

- Str Wa:: Mechanical Strength in the direction of the warp (in N); calculated according to ISO standard 13934-1
- Str We:: Mechanical Strength in the direction of the weft (in N); calculated according to ISO standard 13934-1;
- EI B Wa:: Elongation at break in the direction of the warp (as %); calculated according to ISO standard 13934-1;
- EI B We:: Elongation at break in the direction of the weft (as %); calculated according to ISO standard 13934-1;
- EI Wa 50 N:: Elongation at 50 N in the direction of the warp (as %); calculated according to ISO standard 13934-1;
- EI We 50 N:: Elongation at 50 N in the direction of the weft (as %); calculated according to ISO standard 13934-1.

In a further step, the knit it is subjected to an oxidation step with sodium periodate. This oxidation is carried out by reacting the cellulose in the knit in an aqueous solution of sodium periodate (1:1 molar ratio). The reaction is carried out in the dark for 20 hours at ambient temperature. At the end of the reaction, the knit is washed with water until the pH is about 6-7. The knit is then washed with pure isopropanol.

### EXAMPLE 3: Preparation of a knit according to the present disclosure

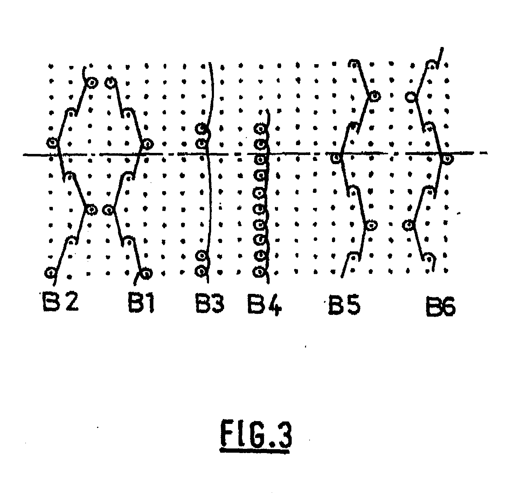

A knit is produced with the same yarns, the same threadings of bars B1 to B6 and according to the same process as in Example 1, but with the difference that bar B4 has the following chart (see Figure 3):
- Bar: B4: 0-1/0-1//.

Bar B4 is threaded with 90 dtex multifilament yarns of regenerated cellulose sold under the name "CUPRO® Cusio" by Bemberg S.p.A., Gozzano, Italy.

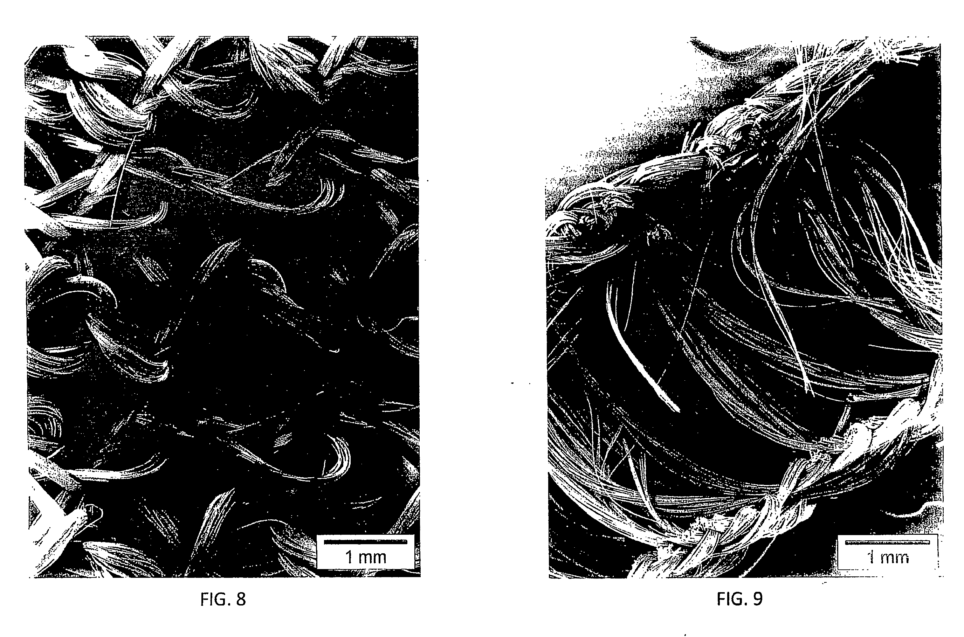

Figure 8 represents a scanning electron microscopy image of one face of such a knit and Figure 9 represents a scanning electron microscopy image of the spacer of such a knit.

Such a knit has the following properties, measured as indicated in the present application:
Weight per surface area (g/m²): 225
Thickness: 4 mm
Three-dimensional porosity: ≥ 80%
Two-dimensional porosity: < 20%

This knit is isoelastic. In particular, it has the following mechanical properties:

| Property | Str Wa | Str We | EI B Wa | EI B We | EI Wa 50 N | EI We 50N |
|---|---|---|---|---|---|---|
| Knit Example 3 | 191 | 156 | 63 | 45 | 36 | 27 |

- Str Wa:: Mechanical Strength in the direction of the warp (in N);
- Str We:: Mechanical Strength in the direction of the weft (in N); calculated according to ISO standard 13934-1;
- EI B Wa:: Elongation at break in the direction of the warp (as %); calculated according to ISO standard 13934-1;
- EI B We:: Elongation at break in the direction of the weft (as %); calculated according to ISO standard 13934-1;
- EI Wa 50 N:: Elongation at 50 N in the direction of the warp (as %); calculated according to ISO standard 13934-1;
- EI We 50 N:: Elongation at 50 N in the direction of the weft (as %); calculated according to ISO standard 13934-1.

The oxidation is carried out as described in Example 1.

### EXAMPLE 4: Coating of the knits of Examples 1 to 3

The knit obtained in Example 1, 2 or 3 is coated in a solution of porcine collagen at 0.8 w/v, by soaking it in the solution, spin-drying it and leaving it to dry under a laminar flow. This cycle of processes is repeated up to two times in order to obtain covering of the yarns.

The collagen used is porcine collagen type I, extracted from porcine dermis by solubilization at acidic pH or by digestion with pepsin, and purified by saline precipitations according to known techniques.

Dry collagen fibres obtained by precipitation of an acid solution of collagen by adding NaCl, and then washing and drying of the precipitate obtained with aqueous solutions of acetone having an increasing concentration of 80% to 100%, are preferably used.

At the end of the coating, the collagen deposited on the knit is crosslinked with glutaraldehyde at 0.5% w/v (aqueous solution of glutaraldehyde at 25%, w/v, sold by the company Fluka), at neutral pH (pH between 6.5 and 7.5), for 2 hours, and is then reduced with sodium borohydride. The reagents used are removed by washing the knit with several water baths.

The crosslinking of the collagen deposited on the knit can alternatively be carried out at the end of each coating cycle.

### Application of a film to one face of the knit:

The coated knit obtained above is subsequently coated with an oxidized collagen film as described in Example 2 of US 6, 391, 939.

A concentrated sterile solution of PEG 4000 (polyethylene glycol having a molecular weight of 4000 D, for example sold by the company Fluka under the trade name PEG 4000) and glycerol is added to a solution of oxidized collagen (obtained by oxidation of porcine collagen) at 3% w/v, so as to obtain a final composition having a PEG 4000 concentration of 1% w/v and a glycerol concentration of 0.6% w/v. The pH of the solution is adjusted to 7.0 by adding a concentrated solution of sodium hydroxide. The volume of the solution is then adjusted with sterile water so as to obtain final concentrations of collagen, of PEG 4000 and of glycerol of 2.7% w/v, 0.9% w/v and 0.54% w/v, respectively. The solution is then spread out so as to form a thin sheet with a density of 0.133 g/cm² on a flat hydrophobic support of polyvinyl chloride or polystyrene type. The surface is then exposed to a stream of sterile air at ambient temperature for just under one hour. The implant obtained above is then applied carefully to the gelled sheet of oxidized collagen above. The whole is exposed to a stream of sterile air at ambient temperature until complete evaporation in about 18 hours.

A knit that is particularly suitable for wall reinforcement and for the prevention of post-surgical adhesions is obtained.

### EXAMPLE 5: Coating of the knits of Examples 1 to 3 :

Knits obtained as in Examples 1 to 3 are coated with chitosan in a single step. Each knit is coated in a 1% chitosan solution (degree of acetylation: 50%; high molecular weight chitosan, extract of chitosan, Mahtani Chitosan Pvt Ltd), by spraying it with the chitosan solution, until the knit has been completely wetted. Each knit is then dried at +50°C. This cycle of processes is repeated up to four times in order to obtain coating of the yarns.

## Claims

1. A bioresorbable three-dimensional prosthetic knit comprising a first face and a second face, said first face and said second face being opposite and separated from one another by the thickness of said knit and being connected to one another by a spacer, said first face and said second face being porous, said first face and said second face comprising yarns made of poly(lactic acid) (PLA), **characterized in that** said spacer comprises yarns made of poly(lactic acid) (PLA) and yarns made of regenerated and oxidized cellulose, said knit exhibiting at least two-speed bioresorption kinetics once implanted.

2. A knit according to Claim 1 wherein said first face and said second face are identical.

3. A knit according to Claim 1 or 2 wherein the yarns made of regenerated and oxidized cellulose define a first network of yarns, said first network having a first density.

4. A knit according to any one of Claims 1 to 3 wherein the yarns made of poly(lactic acid) (PLA) define a second network of yarns, this second network having a second density.

5. A knit according to Claims 3 and 4 wherein said first network and said second network define pores which are interconnected with one another.

6. A knit according to any one of claims 1 to 5 wherein the yarns made of poly(lactic acid) (PLA) that are used for the spacer are monofilament yarns.

7. A knit according to any one of claims 1 to 6 wherein the yarns made of regenerated and oxidized cellulose are multifilament yarns.

8. A knit according to any one of claims 1 to 7 wherein said knit has a mechanical strength in the longitudinal direction, measured according to ISO standard 13934-1, ranging from 50 to 300 N, preferably ranging from 100 to 250 N, said knit has a mechanical strength in the transverse direction, measured according to ISO standard 13934-1, ranging from 50 to 300 N, preferably ranging from 75 to 200 N, said knit has an elongation at 50 N in the longitudinal direction, measured according to ISO standard 13934-1, ranging from 10% to 50%, said knit has an elongation at 50 N in the transverse direction, measured according to ISO standard 13934-1, ranging from 10% to 50%.

## Patentansprüche

1. Bioresorbierbares dreidimensionales prothetisches Maschengewebe, umfassend eine erste Fläche und eine zweite Fläche, wobei die erste Fläche und die zweite Fläche einander gegenüberliegen und durch die Dicke des Maschengewebes voneinander getrennt und durch einen Abstandhalter miteinander verbunden sind, wobei die erste Fläche und die zweite Fläche porös sind, wobei die erste Fläche und die zweite Fläche Garne aus Poly(milchsäure) (PLA) umfassen, **dadurch gekennzeichnet, dass** der Abstandhalter Garne aus Poly(milchsäure) (PLA) und Garne aus regenerierter und oxidierter Cellulose umfasst, wobei das Maschengewebe nach Implantation zumindest 2-Speed-Bioresorption-Kinetik aufweist.

2. Maschengewebe nach Anspruch 1, wobei die erste Fläche und die zweite Fläche identisch sind.

3. Maschengewebe nach Anspruch 1 oder 2, wobei die aus regenerierter und oxidierter Cellulose gebildeten Garne ein erstes Garnnetzwerk definieren, wobei das erste Netzwerk eine erste Dichte aufweist.

4. Maschengewebe nach einem der Ansprüche 1 bis 3, wobei die aus Poly(milchsäure) (PLA) gebildeten Garne ein zweites Garnnetzwerk definieren, wobei das zweite Netzwerk eine zweite Dichte aufweist.

5. Maschengewebe nach Anspruch 3 oder 4, wobei das erste Netzwerk und das zweite Netzwerk Poren definieren, die miteinander verbunden sind.

6. Maschengewebe nach einem der Ansprüche 1 bis 5, wobei die aus Poly(milchsäure) (PLA) gebildeten Garne, die für den Abstandhalter verwendet werden, Monofilamentgarne sind.

7. Maschengewebe nach einem der Ansprüche 1 bis 6, wobei die aus regenerierter und oxidierter Cellulose gebildeten Garne Multifilamentgarne sind.

8. Maschengewebe nach einem der Ansprüche 1 bis 7, wobei das Maschengewebe eine mechanische Festigkeit in Längsrichtung gemessen gemäß ISO-Standard 13934-1 im Bereich von 50 bis 300 N, vorzugweise im Bereich von 100 bis 250 N, aufweist, wobei das Maschengewebe eine mechanische Festigkeit in Querrichtung gemessen gemäß ISO-Standard 13934-1 im Bereich von 50 bis 300 N, vorzugsweise im Bereich von 75 bis 200 N, aufweist, wobei das Maschengewebe eine Dehnung bei 50 N in Längsrichtung gemessen gemäß ISO-Standard 13934-1 im Bereich von 10 % bis 50 % aufweist, wobei das Maschengewebe eine Dehnung bei 50 N in Querrichtung gemessen gemäß ISO-Standard 13934-1 im Bereich von 10 % bis 50 % aufweist.

## Revendications

1. Tricot prothétique tridimensionnel biorésorbable comprenant une première face et une deuxième face, ladite première face et ladite deuxième face étant opposées et séparées l'une de l'autre par l'épaisseur dudit tricot et étant reliées l'une à l'autre par un élément d'espacement, ladite première face et ladite deuxième face étant poreuses, ladite première face et ladite deuxième face comprenant des fils constitués d'acide polylactique (PLA), **caractérisé en ce que** ledit élément d'espacement comprend des fils constitués d'acide polylactique (PLA) et des fils constitués de cellulose régénérée et oxydée, ledit tricot présentant au moins une cinétique de biorésorption à deux vitesses une fois implanté.

2. Tricot selon la revendication 1, dans lequel ladite première face et ladite deuxième face sont identiques.

3. Tricot selon la revendication 1 ou 2, dans lequel les fils constitués de cellulose régénérée et oxydée définissent un premier réseau de fils, ledit premier réseau ayant une première densité.

4. Tricot selon l'une quelconque des revendications 1 à 3, dans lequel les fils constitués d'acide polylactique (PLA) définissent un deuxième réseau de fils, ce deuxième réseau ayant une deuxième densité.

5. Tricot selon les revendications 3 et 4, dans lequel ledit premier réseau et ledit deuxième réseau définissent des pores qui sont interconnectés les uns avec les autres.

6. Tricot selon l'une quelconque des revendications 1 à 5, dans lequel les fils constitués d'acide polylactique (PLA) qui sont utilisés pour l'élément d'espacement sont des fils monofilaments.

7. Tricot selon l'une quelconque des revendications 1 à 6, dans lequel les fils constitués de cellulose régénérée et oxydée sont des fils multifilaments.

8. Tricot selon l'une quelconque des revendications 1 à 7, dans lequel ledit tricot a une résistance mécanique dans la direction longitudinale, mesurée selon la norme ISO 13934-1, qui va de 50 à 300 N, de préférence qui va de 100 à 250 N, ledit tricot a une résistance mécanique dans la direction transversale, mesurée selon la norme ISO 13934-1, qui va de 50 à 300 N, de préférence qui va de 75 à 200 N, ledit tricot a un allongement à 50 N dans la direction longitudinale, mesuré selon la norme ISO 13934-1, qui va de 10 % à 50 %, ledit tricot a un allongement à 50 N dans la direction transversale, mesuré selon la norme ISO 13934-1, qui va de 10 % à 50 %.
